# EUROPEAN PATENT SPECIFICATION

(11) **EP 2 077 892 B1**
(45) Date of publication and mention of the grant of the patent: **24.12.2014**
(21) Application number: 06801749.0
(22) Date of filing: 16.08.2006
(51) Int. Cl.: A61N 1/36, A61N 1/05

(54) **AUTOMATIC FITTING FOR A VISUAL PROSTHESIS**
AUTOMATISCHE ARMATUR FÜR EINE SEHPROTHESE
RACCORD AUTOMATIQUE POUR PROTHÈSE VISUELLE

(43) Date of publication of application: 15.07.2009
(60) Divisional application: 14192904.2
(73) Proprietor: Second Sight Medical Products, Inc., Sylmar, CA 91342 (US)
(72) Inventor: GREENBERG, Robert, J., Los Angeles, CA 90068 (US); WILLIAMSON, Richard, Santa Monica, CA 90403 (US)
(74) Representative: Carter, Stephen John
(86) International application number: PCT/US2006/032157
(87) International publication number: WO 2008/020847

(56) References cited:
- WO-A-00/56393
- WO-A-01/91852
- WO-A2-2006/089155
- US-A1- 2004 236 389

## Description

### Field of the Invention

The present invention is generally directed to neural stimulation and more specifically to adjusting neural stimulation levels for artificial vision.

### Background of the Invention

In 1755 LeRoy passed the discharge of a Leyden jar through the orbit of a man who was blind from cataract and the patient saw "flames passing rapidly downwards." Ever since, there has been a fascination with electrically elicited visual perception. The general concept of electrical stimulation of retinal cells to produce these flashes of light or phosphenes has been known for quite some time. Based on these general principles, some early attempts at devising a prosthesis for aiding the visually impaired have included attaching electrodes to the head or eyelids of patients. While some of these early attempts met with some limited success, these early prosthetic devices were large, bulky and could not produce adequate simulated vision to truly aid the visually impaired.

In the early 1930's, Foerster investigated the effect of electrically stimulating the exposed occipital pole of one cerebral hemisphere. He found that, when a point at the extreme occipital pole was stimulated, the patient perceived a small spot of light directly in front and motionless (a phosphene). Subsequently, Brindley and Lewin (1968) thoroughly studied electrical stimulation of the human occipital (visual) cortex. By varying the stimulation parameters, these investigators described in detail the location of the phosphenes produced relative to the specific region of the occipital cortex stimulated. These experiments demonstrated: (1) the consistent shape and position of phosphenes; (2) that increased stimulation pulse duration made phosphenes brighter; and (3) that there was no detectable interaction between neighboring electrodes which were as close as 2.4 mm apart.

As intraocular surgical techniques have advanced, it has become possible to apply stimulation on small groups and even on individual retinal cells to generate focused phosphenes through devices implanted within the eye itself. This has sparked renewed interest in developing methods and apparatuses to aid the visually impaired. Specifically, great effort has been expended in the area of intraocular retinal prosthesis devices in an effort to restore vision in cases where blindness is caused by photoreceptor degenerative retinal diseases such as retinitis pigmentosa and age related macular degeneration which affect millions of people worldwide.

Neural tissue can be artificially stimulated and activated by prosthetic devices that pass pulses of electrical current through electrodes on such a device. The passage of current causes changes in electrical potentials across visual neuronal membranes, which can initiate visual neuron action potentials, which are the means of information transfer in the nervous system.

Based on this mechanism, it is possible to input information into the nervous system by coding the information as a sequence of electrical pulses which are relayed to the nervous system via the prosthetic device. In this way, it is possible to provide artificial sensations including vision.

One typical application of neural tissue stimulation is in the rehabilitation of the blind. Some forms of blindness involve selective loss of the light sensitive transducers of the retina. Other retinal neurons remain viable, however, and may be activated in the manner described above by placement of a prosthetic electrode device on the inner (toward the vitreous) retinal surface (epiretinal). This placement must be mechanically stable, minimize the distance between the device electrodes and the visual neurons, and avoid undue compression of the visual neurons.

In 1986, Bullara (US Pat. No. 4,573,481) patented an electrode assembly for surgical implantation on a nerve. The matrix was silicone with embedded iridium electrodes. The assembly fit around a nerve to stimulate it.

Dawson and Radtke stimulated cat's retina by direct electrical stimulation of the retinal ganglion cell layer. These experimenters placed nine and then fourteen electrodes upon the inner retinal layer (i.e., primarily the ganglion cell layer) of two cats. Their experiments suggested that electrical stimulation of the retina with 30 to 100 uA current resulted in visual cortical responses. These experiments were carried out with needle-shaped electrodes that penetrated the surface of the retina (see also US Pat. No. 4,628,933 to Michelson).

The Michelson '933 apparatus includes an array of photosensitive devices on its surface that are connected to a plurality of electrodes positioned on the opposite surface of the device to stimulate the retina. These electrodes are disposed to form an array similar to a "bed of nails" having conductors which impinge directly on the retina to stimulate the retinal cells. US Patents 4,837,049 to Byers describes spike electrodes for neural stimulation. Each spike electrode pierces neural tissue for better electrical contact. US Patent 5,215,088 to Norman describes an array of spike electrodes for cortical stimulation. Each spike pierces cortical tissue for better electrical contact.

The art of implanting an intraocular prosthetic device to electrically stimulate the retina was advanced with the introduction of retinal tacks in retinal surgery. De Juan, et al. at Duke University Eye Center inserted retinal tacks into retinas in an effort to reattach retinas that had detached from the underlying choroid, which is the source of blood supply for the outer retina and thus the photoreceptors. See, e.g., E. de Juan, et al., 99 Am. J. Ophthalmol. 272 (1985). These retinal tacks have proved to be biocompatible and remain embedded in the retina, and choroid/sclera, effectively pinning the retina against the choroid and the posterior aspects of the globe. Retinal tacks are one way to attach a retinal array to the retina. US Patent 5,109,844 to de Juan describes a flat electrode array placed against the retina for visual stimulation. US Patent 5,935,155 to Humayun describes a retinal prosthesis for use with the flat retinal array described in de Juan.

In addition to the electrode arrays described above, there are several methods of mapping a high resolution camera image to a lower resolution electrode array. US Patent 6,400,989 to Eckmiller describes spatio-temporal filters for controlling patterns of stimulation in an array of electrodes.

Each person's response to neural stimulation differs. In the case of retinal stimulation, a person's response varies from one region of the retina to another. In general, the retina is more sensitive closer to the fovea. Any stimulation, less than the threshold of perception, is ineffective. Stimulation beyond a maximum level will be painful and possibly dangerous to the patient. It is therefore, important to map any video image to a range between the minimum and maximum for each individual electrode. With a simple retinal prosthesis, it is possible to adjust the stimulation manually by stimulating and questioning the patient. As resolution increases, it is tedious or impossible to adjust each electrode by stimulating and eliciting a patient response.

A manual method of fitting or adjusting the stimulation levels of an auditory prosthesis is described in US patent 4,577,641, Hochmair et al. Hochmair adjusts the auditory prosthesis by having a user compare a received signal with a visual representation of that signal.

A more automated system of adjusting an auditory prosthesis using middle ear reflex and evoked potentials is described in US patent 6,157,861, Faltys et al. An alternate method of adjusting an auditory prosthesis using the stapedius muscle is described in US Patent 6,205,360, Carter et al. A third alternative using myogenic evoked response is disclosed in US Patent 6,415,185, Maltan.

US Patent 6,208,894, Schulman describes a network of neural stimulators and recorders implanted throughout the body communicating wirelessly with a central control unit. US Patent 6,522,928, Whitehurst, describes an improvement on the system described in Schulman using function electro stimulation also know as adaptive delta modulation to communicate between the implanted devices and the central control unit.

WO 01/91852 A2 discloses a visual stimulator comprising a variable output stimulator coupled to an electrode of an electrode array, wherein the electrode array is suitable for contact with retinal tissue. A video camera is used to provide input to a video processor which outputs the stimulation signals. The stimulator further comprises a neural activity recorder coupled to an electrode suitable for contact with neural tissue; a control device for controlling said stimulator in response to said recorder; and a memory for storing data from said recorder. Neural activity is recorded in order to emulate jittering of the eye, or in order to track eye movement for translation of the video image.

WO2006/089155 A2, which is a document falling under Article 54(3) EPC, discloses a visual stimulator comprising a variable output stimulator coupled to an electrode of an electrode array, wherein the electrode array is suitable for contact with retinal tissue; a neural activity recorder coupled to an electrode suitable for contact with neural tissue; a control device for controlling said stimulator in response to said recorder; and a memory for storing data from said recorder, wherein said memory stores a minimum and a maximum stimulation level, wherein said minimum stimulation level is the lowest stimulation level where neural activity is detected. The stimulator further comprises a return electrode distant from the retina.

The greatest dynamic range is achieved by setting the minimum stimulation at the threshold of perception and the maximum stimulation level approaching the pain threshold. It is unpleasant for a patient to first concentrate to detect the minimum perception and then be subjected to stimulation near the threshold of pain.

The human retina includes about four million individual photoreceptors. An effective visual prosthesis may include thousands of electrodes. An automated system is needed to adjust individual electrodes in a visual prosthesis for maximum benefit without the need for patient interaction in a long and difficult process.

### Summary of the Invention

The invention is defined in the appended claims.

A method of automatically adjusting a retinal electrode array to the neural characteristics of an individual patient is disclosed. By recording neural response to a predetermined input stimulus, one can alter that input stimulus to the needs of an individual patient. A minimum input stimulus is applied to a patient, followed by recording neural response in the vicinity of the input stimulus. By alternating stimulation and recording at gradually increasing levels, one can determine the minimum input that creates a neural response, thereby identifying the threshold stimulation level. One can further determine a maximum level by increasing stimulus until a predetermined maximum neural response is obtained.

The invention will be best understood from the following description when read in conjunction with the accompanying drawings.

### Brief Description of the Drawings

**FIG. 1** depicts the preferred retinal prosthesis for implementing the present invention.
**FIG. 2** is a flow chart showing the process of auto fitting an electrode array.
**FIG. 3** depicts a block diagram of the retinal prosthesis electronic control unit.
**FIG. 4** is a graph depicting a typical neural response to electrical input.
**FIG. 5** depicts an alternate retinal prosthesis using cortical recording.
**FIG. 6** depicts an alternate retinal prosthesis using iris recording.

### Detailed Description of the Preferred Embodiments

The following description is of the best mode presently contemplated for carrying out the invention. This description is not to be taken in a limiting sense, but is made merely for the purpose of describing the general principles of the invention. The scope of the invention should be determined with reference to the claims.

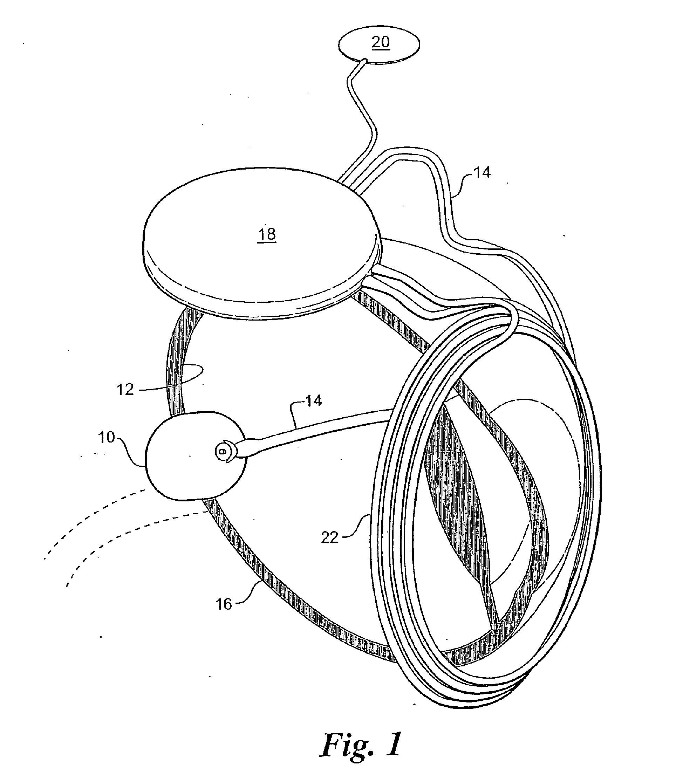

Figure 1 shows the preferred retinal prosthesis. A stimulating electrode array **10** is placed against the outer surface of a retina **12** (epiretinally). A cable **14** pierces a sclera **16** and attaches to an electronic control unit **18.** The electronic control unit is attached to the sclera and moves with the sclera. In an embodiment not forming part of the present invention, a return electrode **20** may be placed outside the sclera and distant from the retina **12**. Alternatively and in accordance with the present invention, electrodes in the electrode array **10** may be used as return electrodes. Electricity travels through the body between the stimulating electrode array **10** and return electrode **20,** to complete an electrical circuit. The stimulating electrode array **10** is a plurality of tiny electrodes. Each electrode on the stimulating electrode array **10** is as small as possible to maximize the effect of electrical current on the retina, and to fit the maximum number of electrodes on the retina. The return electrode **20,** if used, may be quite large by comparison. A coil **22** surrounds the sclera just inside the conjunctiva and acts as an antenna to send and receive data from an external unit (not shown). A matching coil is mounted in a pair of glasses along with a camera for collecting a video image. Power to operate the control unit may also be provided through the coil **22.**

The electronics described herein may be in the electronics control unit **18** or mounted externally and communicate through the coil **22.** An external solution may initially be simpler and less expensive. With improvements in integrated circuits, it will be cost effective to include all of the control functions described herein within the control unit **18.** An entirely implanted solution would greatly reduce the time required to complete the fitting process.

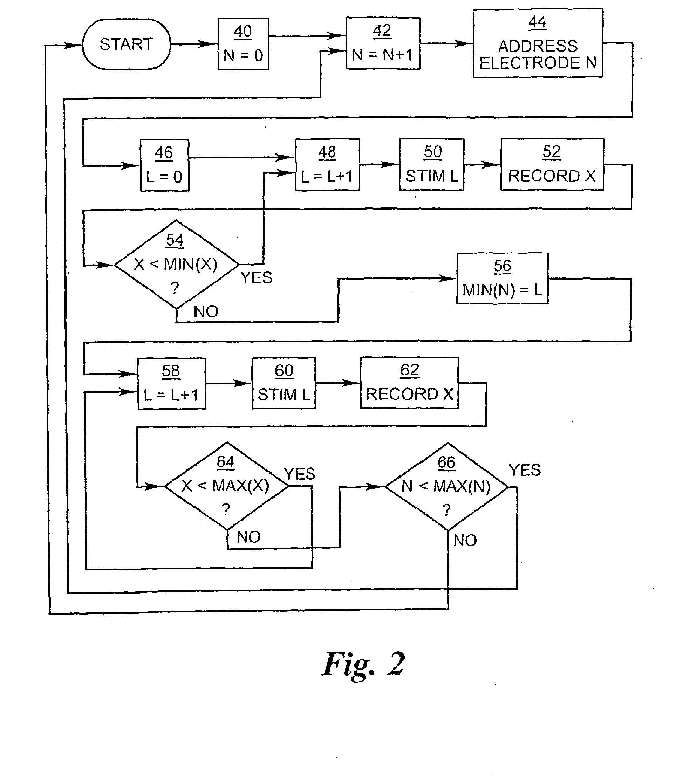

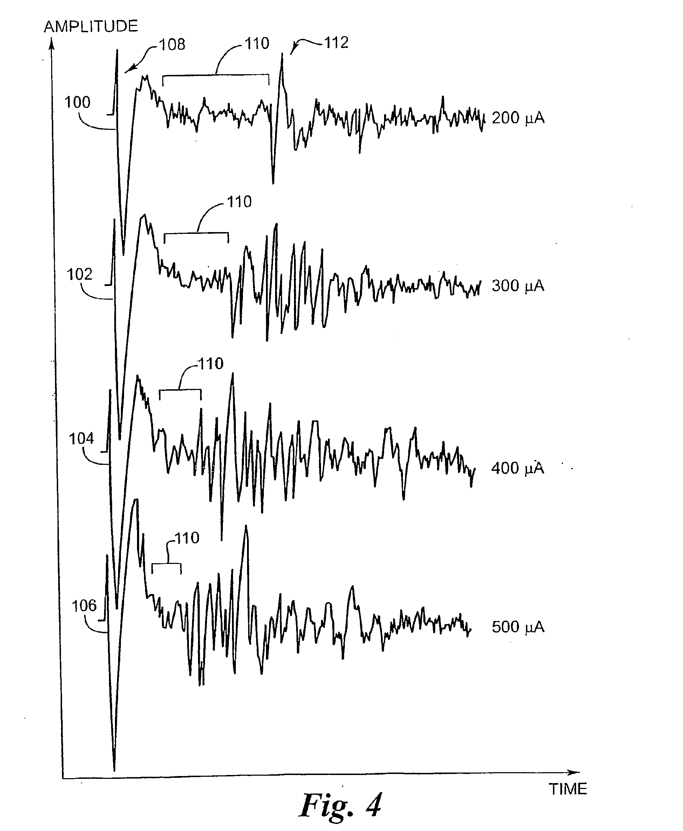

Figure 2 is a flow chart of the automatic fitting sequence. In the flow chart, the value N is the current (or selected) electrode, X is the neural activity recorded, and L is the level of stimulation. First N is set to 0 **40** and then incremented **42.** The first electrode, electrode N, is addressed **44.** The stimulation level is set to zero **46,** and then incremented **48.** The neural tissue is stimulated at the minimum level **50.** The stimulation is immediately followed by a recording of activity in the neural tissue **52.** Alternatively, recording can be done simultaneously by an adjacent electrode. If recording is done simultaneously, one must be careful to distinguish between neural activity and electrical charge from the stimulating electrode. The neural response follows stimulation (see figure 4). Simultaneous stimulation and recording requires that the recording phase be longer than the stimulation phase. If so, the stimulation and neural response can be separated digitally. If the recorded neural activity is less than a predetermined level **54,** the stimulation level is increased and steps **48** - **54** are repeated.

In most cases, the preset minimum level is any measurable neural activity. However, perception by the patient is the determining factor. If neural activity is detected and the patient reports no perception, the minimum level must be set higher. Once minimum neural activity is recorded, the stimulation level is saved in memory **56.** The level is then further increased **58** and stimulation is repeated **60.** Again stimulation is immediately followed by recording neural activity **62.** If a predetermined maximum level has not been reached, steps **58** - **64** are repeated until the predetermined maximum stimulation level is obtained. Once the predetermined maximum stimulation level is obtained, steps **42** - **64** are repeated for the next electrode. The process is continued until a minimum and maximum stimulation level is determined for each electrode **66.**

The maximum stimulation level borders on discomfort for the patient. Because the automatic fitting process is automated, high levels of stimulation are only applied for a few microseconds. This significantly decreases the level of discomfort for the patient compared with stimulating long enough to elicit a response from the patient.

The fitting process is described above as an incremental process. The fitting process may be expedited by more efficient patterns. For example changes may be made in large steps if it the detected response is significantly below the desired response, followed by increasingly small steps as the desired response draws near. The system can jump above and below the desired response dividing the change by half with each step.

Often, neural response in a retina is based, in part, geographically. That is, neurons closer to the fovea require less stimulation than neurons farther from the fovea. Hence once a stimulation is level is set for an electrode, one can presume that the level will be similar for an adjacent electrode. The fitting process may be expedited by starting at a level near the level set for a previously fit adjacent electrode.

Automating the fitting process has many advantages. It greatly expedites the process reducing the efforts of the patient.and clinician. Further, the automated process is objective. Patient responses are subjective and may change over time due to fatigue. In some cases, a patient may not be able to provide the required responses due to age, disposition, and/or limited metal ability.

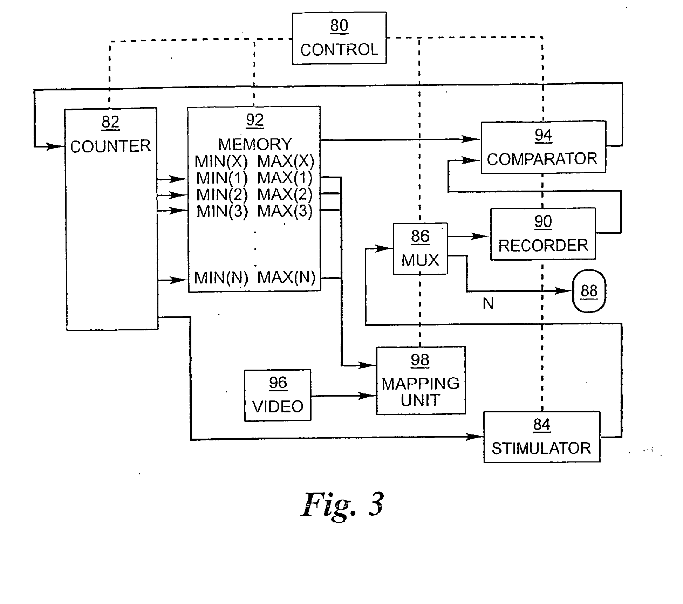

Figure 3 depicts a block diagram of the control unit. The block diagram is a functional diagram. Many of the functional units would be implemented in a microprocessor. A control unit **80** sets and increments a counter **82** to control the stimulation level of the stimulator **84.** The stimulation signal is multiplexed in MUX **86** to address individual electrodes **88.** After each stimulation, the addressed electrode returns a neural activity signal to a recorder **90.** The signal is compared to the stored minimum or maximum level (stored in a memory **92)** in a comparator **94.** After programming, a signal from a video source **96,** or other neural stimulation source, is adjusted in a mapping unit **98,** in accordance with the minimum and maximum levels stored in the memory **92.** The adjusted signal is sent to the stimulator **84,** which in synchronization with MUX **86** applies the signal to the electrodes **88.** The electronics for the control unit could be external or within the implanted prosthesis.

Figure 4 is a graphical representation of the neural response to electrical stimulus. This figure is derived from actual recordings of a frog retina. Response in a human retina will be similar. The vertical axis is current while the horizontal axis is time. Four curves **100 - 106** show the response at varying input current levels. An input pulse **108,** is followed by a brief delay **110,** and a neural response **112.** Hence, it is important to properly time the detecting function. Either the stimulating electrode must be switched to a detecting electrode during the brief delay or detecting must occur on another electrode and continue long enough to record the neural response. It should also be noted that the delay period **110** becomes shorter with increased stimulation current. Hence, the system must switch faster from stimulation mode to detecting mode with increased current. The change in delay time may also be used as an additional indication of neural response. That is, the minimum and maximum may be determined by matching predetermined delay times rather than predetermined output levels. As stimulation increases, it becomes more useful to employ an alternate recording means as described in the following alternate embodiments.

In a first alternate embodiment, the recording electrode may be a cortical electrode mounted on or near the visual cortex. Temporary external electrodes placed on the scalp proximate to the visual cortex may record neural activity in the visual cortex. This allows the system to account for any variations in neural processing between the retina and the visual cortex. It, however, requires electrodes either implanted in the visual cortex or placed temporarily near the visual cortex. This alternate embodiment may be combined with the preferred embodiment by first using cortical electrodes to perform an initial fitting of the prosthesis in a clinic. Thereafter, retinal recording may be used to readjust the prosthesis for any changes over time.

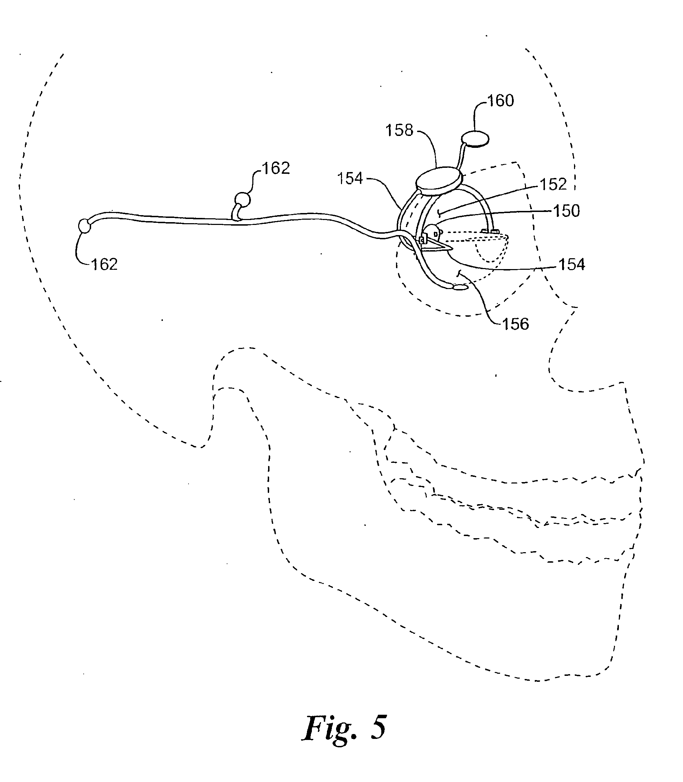

Figure 5 shows the first alternate retinal prosthesis. A stimulating electrode array **150** is placed against the outer surface of a retina **152** (epiretinally). A cable **154** pierces a sclera **156** and attaches to an electronic control unit **158.** A return electrode **160** may be placed distant from the retina **152.** The stimulating electrode array **150** is a plurality of tiny electrodes. One or more recording electrodes **162** are placed in near the visual cortex. The recording electrodes may be temporary external electrodes, implanted electrodes under the scalp, or electrodes implanted within the visual cortex.

In a second alternate embodiment, the recording electrode may be either implanted in the iris, or placed externally near the iris. The iris responds to light, or the perception of light. In response to an increase in electrical stimulation the iris will contract because the body perceives an increase in light entering the eye. Conversely, the iris expands in response to a decrease in electrical stimulation. While the response of the iris is relatively slow, the neurological signals initiating a change in the iris respond quickly. Measuring these signals may provide alternate feed back as to the body's response to the electrical stimulus. Alternatively, an optical device aimed at the eye may detect the movement of the iris.

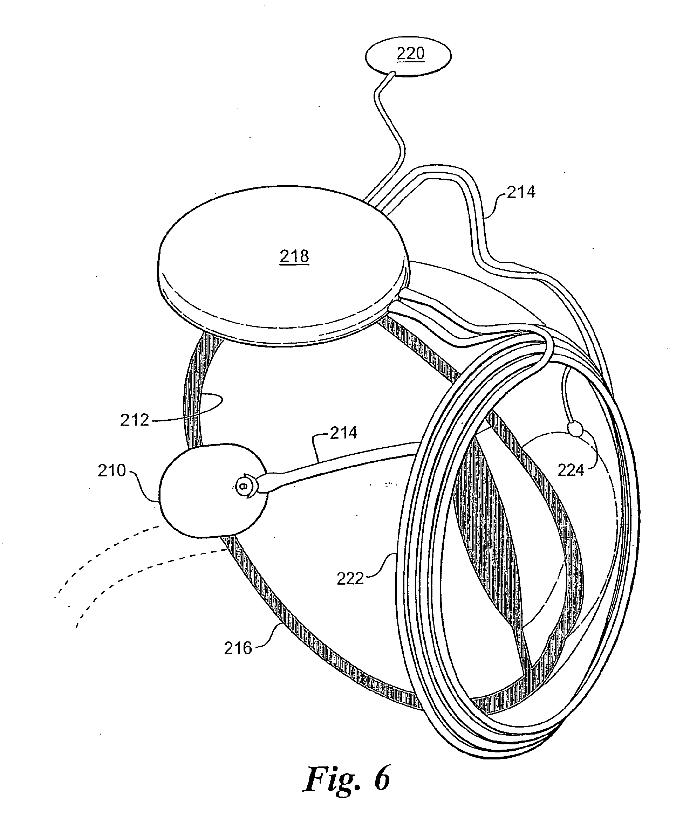

Figure 6 shows the second alternate retinal prosthesis. A stimulating electrode array **210** is placed against the outer surface of a retina **212** (epiretinally). A cable **214** pierces a sclera **216** and attaches to an electronic control unit **218.** A return electrode **220** may be placed distant from the retina **212.** The stimulating electrode array **210** is a plurality of tiny electrodes. A recording electrode **224** is place in the periphery of the iris sensing electrical stimulus to the iris.

In a third alternate device, which does not form part of the present invention, electroluminescent pigments may be applied to the retina. Electroluminescent pigments cause an individual cell to glow when it fires it neuro-electrical charge. A camera of the type used for retinal photos may detect neural response by detecting the electroluminescent glow of the applied pigment.

While the invention has been described by means of specific embodiments and applications thereof, it is understood that numerous modifications and variations could be made thereto by those skilled in the art without departing from the scope of the invention as defined in the appended claims.

## Claims

1. A visual stimulator comprising:
an electrode array suitable for contact with retinal tissue and wherein electrodes in the electrode array are configured to be used as return electrodes;
a variable output stimulator (84) coupled to an electrode (88) of the electrode array (10);
a neural activity recorder (90) coupled to an electrode suitable for contact with neural tissue;
a control device (80) for controlling said stimulator (84) in response to said recorder (90);
a memory (92) for storing data from said recorder (90),
wherein said memory (92) stores a minimum and a maximum stimulation level,
wherein said minimum stimulation level is the lowest stimulation level where neural activity is detected.

2. The visual stimulator according to claim 1, wherein said neural activity recorder (90) is a visual cortex activity recorder.

3. The visual stimulator according to claim 1, wherein said neural activity recorder (90) is an iris sphincter activity recorder.

4. The visual stimulator according to claim 1, wherein said control device (80) is adapted to stimulate visual neurons between said minimum and said maximum stimulation level according to an external input.

5. The visual stimulator according to claim 4, wherein said external input is a video capture device (96).

6. The visual stimulator according to claim 1, wherein said electrode (88) coupled to said stimulator (84) is adjacent to said electrode coupled to said recorder (90).

7. The visual stimulator according to claim 1, wherein said electrode (88) suitable for contact with neural tissue is suitable for implantation in an eye.

8. A visual prosthesis comprising:
the visual stimulator according to claim 1, wherein the neural activity recorder (90) is coupled to a plurality of electrodes suitable for contact with retinal neurons;
wherein said memory (92) is adapted to store a preset minimum level of neural activity and a preset maximum level of neural activity;
wherein the control device (80) is adapted to determine said minimum level of stimulation as the level causing said minimum level of neural activity and said maximum level of stimulation as the level causing said maximum level of neural activity;
wherein said minimum level of neural activity is the minimum level for which neural activity is detected.

9. The visual prosthesis according to claim 8, being adapted to determine said minimum level of stimulation and said maximum level of stimulation separately for each electrode.

10. The visual prosthesis according to claim 8, further comprising a video capture device (96) coupled to said control device (80) adapted to control said stimulator (84) between said minimum stimulation level and said maximum stimulation level according to a video image.

11. The visual prosthesis according to claim 8, being adapted to recalibrate minimum stimulation level and said maximum stimulation level on a periodic basis.

## Patentansprüche

1. Visueller Stimulator, umfassend
eine Elektrodenanordnung, die für den Kontakt mit Netzhautgewebe geeignet ist, und wobei Elektroden in der Elektrodenanordnung konfiguriert sind, als Rücklaufelektroden verwendet zu werden;
einen variablen Ausgangsstimulator (84), der mit einer Elektrode (88) der Elektrodenanordnung (10) verbunden ist;
eine Nervenaktivitätsaufnahmevorrichtung (90), die mit einer Elektrode verbunden ist, die für den Kontakt mit Nervengewebe geeignet ist;
eine Steuervorrichtung (80) zum Steuern des Stimulators (84) als Antwort auf die Aufnahmevorrichtung (90); und
einen Speicher (92) zum Speichern von Daten aus der Aufnahmevorrichtung (90),
wobei der Speicher (92) einen Mindest- und einen Höchststimulierungspegel speichert, wobei
der Mindeststimulierungspegel der niedrigste Stimulierungspegel ist, bei dem Nervenaktivität detektiert wird.

2. Visueller Stimulator nach Anspruch 1, wobei die Nervenaktivitätsaufnahmevorrichtung (90) eine visuelle Cortexaktivitätsaufnahmevorrichtung ist.

3. Visueller Stimulator nach Anspruch 1, wobei die Nervenaktivitätsaufnahmevorrichtung (90) eine Irisschließmuskelaktivitätsaufnahmevorrichtung ist.

4. Visueller Stimulator nach Anspruch 1, wobei die Steuervorrichtung (80) angepasst ist, visuelle Neuronen zwischen dem Mindest- und dem Höchststimulierungspegel gemäß einer äußeren Eingabe zu stimulieren.

5. Visueller Stimulator nach Anspruch 4, wobei die äußere Eingabe eine Videoaufnahmevorrichtung (96) ist.

6. Visueller Stimulator nach Anspruch 1, wobei die Elektrode (88), die mit dem Stimulator (84) verbunden ist, zu der mit der Aufnahmevorrichtung (90) verbundenen Elektrode benachbart ist.

7. Visueller Stimulator nach Anspruch 1, wobei die Elektrode (88), die für den Kontakt mit Nervengewebe geeignet ist, für die Implantation in ein Auge geeignet ist.

8. Visuelle Prothese, umfassend:
den visuellen Stimulator nach Anspruch 1, wobei die Nervenaktivitätsaufnahmevorrichtung (90) mit einer Vielzahl von Elektroden verbunden ist, die für den Kontakt mit Netzhautneuronen geeignet ist;
wobei der Speicher (92) geeignet ist, einen vorbestimmten Mindestpegel an Nervenaktivität und einen vorbestimmten Höchstpegel an Nerventätigkeit zu speichern; wobei
die Steuervorrichtung (80) geeignet ist, den Mindestpegel an Stimulierung als den Pegel zu erkennen, der den Mindestpegel an Nervenaktivität bewirkt, und den Höchstpegel an Stimulierung als den Pegel zu erkennen, der den Höchstpegel an Nervenaktivität bewirkt; wobei
der Mindestpegel an Nervenaktivität der Mindestpegel ist, für den Nervenaktivität detektiert wird.

9. Visuelle Prothese nach Anspruch 8, wobei diese geeignet ist, den Mindestpegel an Stimulierung und den Höchstpegel an Stimulierung separat für jede Elektrode zu bestimmen.

10. Visuelle Prothese nach Anspruch 8, ferner umfassend eine Videoaufnahmevorrichtung (96), die mit der Steuervorrichtung verbunden ist, die geeignet ist, den Stimulator (84) zwischen dem Mindeststimulierungspegel und dem Höchststimulierungspegel gemäß einem Videobild zu steuern.

11. Visuelle Prothese nach Anspruch 8, wobei diese geeignet ist, den Mindeststimulierungspegel und den Höchststimulierungspegel auf einer periodischen Basis zu rekalibrieren.

## Revendications

1. Stimulateur visuel comprenant :
un réseau d'électrodes pertinent en vue d'un contact avec du tissu rétinien, et dans lequel les électrodes du réseau d'électrodes sont configurées de manière à être utilisées en qualité d'électrodes de référence ;
un stimulateur à sortie variable (84) couplé à une électrode (88) du réseau d'électrodes (10) ;
un enregistreur d'activité neuronale (90) couplé à une électrode pertinente en vue d'un contact avec du tissu neuronal ;
un dispositif de commande (80) destiné à commander ledit stimulateur (84) en réponse audit enregistreur (90) ;
une mémoire (92) destinée à stocker des données en provenance dudit enregistreur (90) ;
dans lequel ladite mémoire (92) stocke un niveau de stimulation minimum et un niveau de stimulation maximum ;
dans lequel ledit niveau de stimulation minimum correspond au niveau de stimulation le plus faible pour lequel une activité neuronale est détectée.

2. Stimulateur visuel selon la revendication 1, dans lequel ledit enregistreur d'activité neuronale (90) est un enregistreur d'activité de cortex visuel.

3. Stimulateur visuel selon la revendication 1, dans lequel ledit enregistreur d'activité neuronale (90) est un enregistreur d'activité sphinctérienne de l'iris.

4. Stimulateur visuel selon la revendication 1, dans lequel ledit dispositif de commande (80) est apte à stimuler des neurones visuels entre ledit niveau de stimulation minimum et ledit niveau de stimulation maximum selon une entrée externe.

5. Stimulateur visuel selon la revendication 4, dans lequel ladite entrée externe correspond à un dispositif de capture vidéo (96).

6. Stimulateur visuel selon la revendication 1, dans lequel ladite électrode (88) couplée audit stimulateur (84) est adjacente à ladite électrode couplée audit enregistreur (90).

7. Stimulateur visuel selon la revendication 1, dans lequel ladite électrode (88) pertinente en vue d'un contact avec le tissu neuronal est pertinente en vue d'une implantation dans un oeil.

8. Prothèse visuelle comprenant :
le stimulateur visuel selon la revendication 1, dans laquelle l'enregistreur d'activité neuronale (90) est couplé à une pluralité d'électrodes pertinentes en vue d'un contact avec des neurones rétiniens ;
dans laquelle ladite mémoire (92) est apte à stocker un niveau minimum prédéfini d'activité neuronale et un niveau maximum prédéfini d'activité neuronale ;
dans laquelle le dispositif de commande (80) est apte à déterminer ledit niveau minimum de stimulation en tant que le niveau occasionnant ledit niveau minimum d'activité neuronale, et ledit niveau maximum de stimulation en tant que le niveau occasionnant ledit niveau maximum d'activité neuronale ;
dans laquelle ledit niveau minimum d'activité neuronale correspond au niveau minimum pour lequel une activité neuronale est détectée.

9. Prothèse visuelle selon la revendication 8, laquelle est apte à déterminer ledit niveau minimum de stimulation et ledit niveau maximum de stimulation séparément pour chaque électrode.

10. Prothèse visuelle selon la revendication 8, comprenant en outre un dispositif de capture vidéo (96) couplé audit dispositif de commande (80) apte à commander ledit stimulateur (84) entre ledit niveau de stimulation minimum et ledit niveau de stimulation maximum selon une image vidéo.

11. Prothèse visuelle selon la revendication 8, laquelle est apte à ré-étalonner ledit niveau de stimulation minimum et ledit niveau de stimulation maximum sur une base périodique.
